# EUROPÄISCHE PATENTANMELDUNG

(11) **EP 0 874 238 A1**
(43) Veröffentlichungstag der Anmeldung: **28.10.1998**
(21) Anmeldenummer: 98104172.6
(22) Anmeldetag: 09.03.1998
(51) Int. Cl.: G01N 29/00, G01N 29/02, G01N 29/20, G01P 5/00, A61B 5/087

(54) **Messvorrichtung zum gleichzeitigen Bestimmen des Flusses einer strömenden Gasmischung und der Konzentration eines spezifischen Gases in der Gasmischung**

(30) Priorität: 21.04.1997 SE 9701477
(71) Anmelder: Siemens-Elema AB, 171 95 Solna 1 (SE)
(72) Erfinder: Wallen, Lars, 163 60 Spanga (SE); Castor, Rolf, 129 42 Hägersten (SE)

(57) **Zusammenfassung**

Es wird eine Meßvorrichtung zum gleichzeitigen Bestimmen des Flusses einer strömenden Gasmischung und der Konzentration eines spezifischen Gases in der Gasmischung beschrieben. Die Meßvorrichtung enthält eine Meßkammer, durch die die Gasmischung strömen kann, einen akustischen Sender (26) zum Erzeugen und Abgeben eines akustischen Signals durch die Meßkammer, einen akustischen Empfänger (28), um ein übertragenes akustisches Signal zu messen, und eine erste, an den akustischen Empfänger (28) angeschlossene Analyseneinheit (36), um den Gasfluß durch die Meßkammer (23) aus dem Einfluß des Gasflusses auf das akustische Signal zu bestimmen, sowie eine zweite, an den akustischen Empfänger (28) angeschlossene Analyseneinheit (38), um die Konzentration des spezifischen Gases in der Gasmischung durch Bestimmen des Einflusses des spezifischen Gases auf das akustische Signal zu bestimmen.

## Beschreibung

Die vorliegende Erfindung betrifft eine Meßvorrichtung gemäß dem Oberbegriff des Anspruchs 1.

Eine derartige Meßvorrichtung ist beispielsweise durch die EP-646 346 bekannt. Die bekannte Meßvorrichtung enthält ein Atmungsrohr, durch das ein Patient bei einer Lungenfunktionsuntersuchung atmen soll. Der Gasfluß durch das Atmungsrohr kann mit Hilfe eines in dem Atmungsrohr angeordneten Ultraschallflußmessers bestimmt werden.

In dem Atmungsrohr in der bekannten Meßvorrichtung ist auch ein optischer Konzentrationsmesser zum Messen der Konzentration einer oder mehrerer Gaskomponenten im Atemgas angeordnet.

Eine gleichzeitige Messung des Flusses einer Gasmischung und der Konzentration einer Gaskomponente in der Gasmischung hat gewisse Vorteile, insbesondere im Zusammenhang mit der Messung von Atemgasen. Der Aufbau mit einem akustischen Flußmesser und einem optischen Konzentrationsmesser macht die Meßvorrichtung jedoch unhandlich und stellt hohe Anforderungen an die Elektronik, um eine Korrelation zwischen den Bestimmungen der verschiedenen Parameter zu erhalten.

Es ist an sich bekannt, die Konzentration eines Gases mit Hilfe von Ultraschall zu bestimmen. Eine derartige Meßvorrichtung wird beispielsweise in der US-5,313,820 beschrieben. In der bekannten Meßvorrichtung wird das Gas so geleitet, daß es eine Meßkammer passiert, durch die wenigstens ein Teil des Gases dazu gebracht wird, turbulent zu strömen und zu wirbeln. Die Gasprobe strömt dadurch um die zentral angeordneten Ultraschallsensoren in der Meßkammer herum. Die Gaskonzentration wird gemessen, indem eine Phasenverschiebung in einem durch das Gas geleiteten akustischen Signals bestimmt wird.

Die Meßvorrichtung ist im wesentlichen dafür vorgesehen, die Konzentration von Sauerstoff in dem Atemgas zu bestimmen. Hierbei ist vorgesehen, daß nur ein Teil des totalen Atemgasflusses zur Bestimmung des Sauerstoffgehalts durch die Meßkammer hindurchgeleitet wird. Die Konstruktion der Meßkammer selbst bringt mit sich, daß der Gasfluß, der hinein in die oder durch die Meßkammer strömt, stark beeinflußt wird. Die bekannte Meßvorrichtung ist daher allein für Konzentrationsmessungen konstruiert und darauf begrenzt.

Eine andere bekannte Meßvorrichtung zur Konzentrationsbestimmung mittels Ultraschall ist in der US-4,616,501 beschrieben. Die andere bekannte Meßvorrichtung umfaßt ein Sensorsystem, bei dem Ultraschall bei einer Mehrzahl unterschiedlicher Frequenzen erzeugt und durch die Probenkammer übertragen wird. Danach wird die Amplitude an verschiedenen Orten in der Probenkammer bei unterschiedlichen Frequenzen gemessen. Die so erhaltenen Meßwerte werden dann mit einem mathematischen Modell verglichen, das für die Amplitudenantwort der akustischen Signale für spezifische Gaszusammensetzungen experimentell ermittelt worden ist. Durch Vergleichen der ermittelten Amplitudenantworten mit den bekannten gespeicherten Amplitudenantworten kann die Konzentration eines spezifischen Gases bestimmt werden. Die Meßvorrichtung ist in erster Linie zum Bestimmen der Konzentration einer Sterilisierungskomponente (Äthylenoxid)in einer Sterilisierungskammer vorgesehen. Die Meßvorrichtung führt eine Methode aus, die kompliziert ist und eine Menge von Ausrüstung und Computerleistung erfordert, um die Amplituden messen zu können und die gemessenen Amplituden mit Hilfe eines Datenmodells vergleichen zu können. Außerdem wird für jedes spezifische Gas, das in einer spezifischen Gasmischung analysiert werden soll, eine umfassende Datensammlung bekannter Konzentrationen in unterschiedlichen Mischungsvariationen benötigt. Die Meßmethode selbst begrenzt deren Anwendungsmöglichkeit auf isolierte Meßkammern ohne passierende Gasflüsse mit variierender Gaszusammensetzung.

Eine Aufgabe der Erfindung ist es, eine Meßvorrichtung gemäß dem Oberbegriff zu schaffen, die auf einfache und elegante Weise gleichzeitig die Bestimmung des Flusses einer strömenden Gasmischung und der Konzentration eines spezifischen Gases in der Gasmischung ermöglicht.

Diese Aufgabe wird gemäß der Erfindung dadurchgelöst, daß die Meßvorrichtung mit einer zweiten, an den akustischen Empfänger angeschlossenen Analyseneinheit ausgestattet ist, um die Konzentration des spezifischen Gases in der Gasmischung durch Bestimmen des Einflusses des spezifischen Gases auf die akustischen Signale zu bestimmen.

Durch Ausnützen des Signals, das sich im Zusammenhang mit der Flußmessung der Gasmischung ergibt, kann die Meßvorrichtung hinsichtlich der Komponenten minimiert und billiger, stabiler und genauer gemacht werden.

Im Prinzip kann die Analyse des übertragenen Signals digital durchgeführt werden, wobei die Information des Flusses und der Konzentration schnell und einfach in Form digitaler Signale erhalten wird, die dann auf bekannte Art und Weise bearbeitet oder dargestellt werden können.

Eine Vielzahl vorteilhafter Weiterentwicklungen der Meßvorrichtung ergeben sich aus den Unteransprüchen zum Patentanspruch 1.

Die Meßvorrichtung gemäß der Erfindung ist besonders dazu geeignet, in Ventilator-/Anästhesiesystemen verwendet zu werden, wobei sie vorzugsweise in der Exspirationsleitung angeordnet wird.

Um zusätzlich exakte und schnelle Messungen bei einem Minimum von Elektronik und Ansätzen mathematischer Modelle zu ermöglichen, kann die Meßvorrichtung alternativ in einer Trachealröhre oder dergleichen angeordnet werden, wodurch Atemgas sowohl während der Inspiration als auch während der Exspiration fließt. Während der Inspiration kann damit der eingeatmete Atemgasfluß gleichzeitig damit bestimmt werden, daß ein Referenzwert für die Gaszusammensetzung in dem eingeatmeten Atemgas bestimmt wird. Während der Exspiration kann dann der ausgeatmete Atemgasfluß gleichzeitig damit gemessen werden, daß Gaskomponenten wie Kohlendioxid einfach bestimmt werden. Die Konzentrationsbestimmung wird in diesem Fall offensichtlich einfach, nachdem der wesentliche Unterschied in der Gaszusammensetzung zwischen dem eingeatmeten Gas und dem ausgeatmeten Gas gerade in dem Kohlendioxidgehalt besteht. Der Kohlendioxidgehalt in dem Ausatmungsgas kann durch Herausfiltern einer spezifischen Frequenz aus dem akustischen Signal und der Bestimmung der Amplitudenveränderungen gemessen werden.

Unter Bezugnahme auf die Figuren soll die Meßvorrichtung gemäß der Erfindung näher beschrieben werden, wobei
Fig. 1 die in einem Ventilatorsystem angeordnete Meßvorrichtung zeigt,
Fig. 2 eine erste Ausführungsform der Meßvorrichtung zeigt,
Fig. 3 eine Steuervorrichtung in der Meßvorrichtung gemäß der Erfindung zeigt,
Fig. 4 eine zweite Ausführungsform der Meßvorrichtung zeigt und
Fig. 5 eine alternative Anordnung der Meßvorrichtung in einem Ventilatorsystem zeigt.

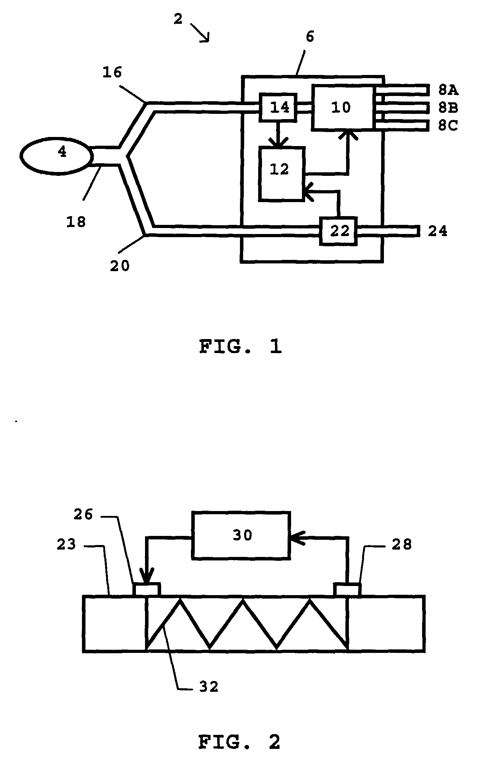

Fig. 1 zeigt ein Ventilatorsystem 2, das einen Patienten 4 mit einem Atemgas versieht. Das Ventilatorsystem 2 umfaßt eine Ventilatoreinheit 6, an die über einen ersten Gasanschluß 8A, einen zweiten Gasanschluß 8B und einen dritten Gasanschluß 8C ein oder mehrere Gas(e) angeschlossen werden kann (können). Das angeschlossene Gas oder die angeschlossenen Gase werden in einer Mischeinheit 10 gemischt und auch so geregelt, daß das fertig gemischte Gas eine vorbestimmte Zusammensetzung erhält sowie einem gewissen Druck- und Flußmuster folgt. Die Parameter werden über eine Steuervorrichtung 12 gesteuert. Die fertige Gasmischung wird dem Patienten 4 über eine Inspirationsleitung 16 und einen Patientenschlauch 18, beispielsweise einen Trachealtubus, zugeführt. Ausgeatmetes Gas vom Patienten geht über den Patientenschlauch 18 und eine Exspirationsleitung 20 zurück zur Ventilatoreinheit 6, wonach es über einen Evakuierungsausgang 24 evakuiert wird. Das ausgeatmete Gas kann auf bekannte Weise hinsichtlich Druck in der Ventilatoreinheit 6 (in der Figur nicht dargestellt) geregelt werden. In dem Exspirationsteil ist eine Meßvorrichtung 22 zur gleichzeitigen Bestimmung des Flusses und der Konzentration angeordnet.

Die Meßvorrichtung 22 umfaßt, wie aus der Fig. 2 hervorgeht, eine Meßkammer 23, durch die das gesamte ausgeatmete Gas strömt, einen akustischen Sender 26, beispielsweise einen piezoelektrischen Kristall, einen akustischen Empfänger 28, der ebenfalls aus einem piezoelektrischen Kristall bestehen kann, und eine Analysenvorrichtung 30. Die Analysenvorrichtung kann hierbei in die Meßeinheit 22 selbst integriert sein oder beispielsweise in der Steuereinheit 12 in der Ventilatoreinheit 6 angeordnet sein.

Der akustische Sender 26 gibt ein akustisches Signal 32 ab, das über eine Anzahl Reflexionen an den Wänden der Meßkammer 23 durch die strömende Gasmischung übertragen wird und dann aufden akustischen Empfänger 28 trifft, der das übertragene akustische Signal empfängt. Um die Reflexionen zu erhalten, kann die Ausbildung der Meßkammer 23 auf eine der bekannten Weisen, die es auf dem Gebiet der akustischen Flußmessung gibt, erfolgen.

Die Analysenvorrichtung 30 bearbeitet das empfangene Signal und bestimmt aus diesem den Fluß der Gasmischung sowie die Konzentration zumindest einer in der Gasmischung enthaltenen Gaskomponente, beispielsweise Kohlenoxid.

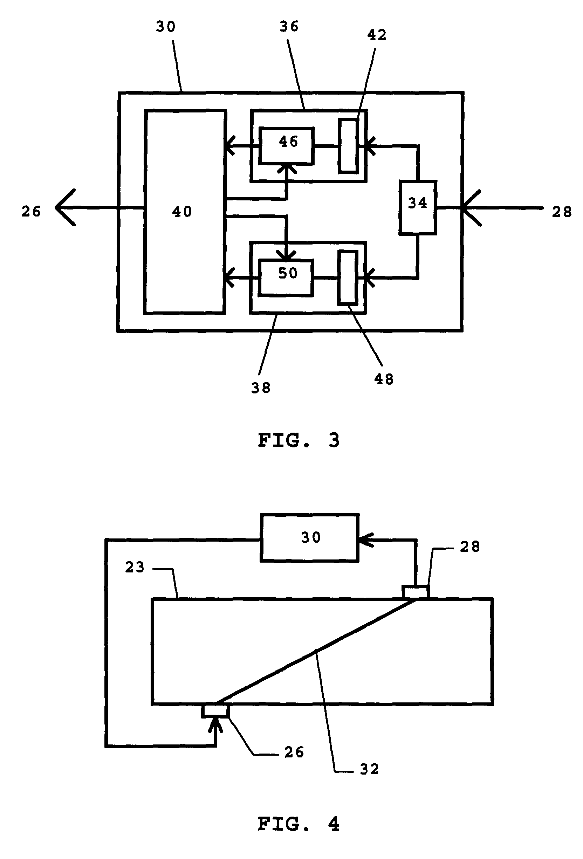

In Fig. 3 wird eine Ausführungsform der Analysenvorrichtung 30 gezeigt. Das gemessene akustische Signal wird in dem akustischen Empfänger 28 in ein elektrisches Signal umgewandelt, das auf einen Vorverstärker 34 in der Analysenvorrichtung 30 überführt wird. Das vorverstärkte Signal wird teilweise zu einer ersten Analyseneinheit 36 und teilweise zu einer zweiten Analyseneinheit 38 geleitet. In der ersten Analyseneinheit 36 wird der momentane Fluß bestimmt und diese Angaben werden kontinuierlich zu einer Berechnungseinheit 40 übertragen. Auf entsprechende Weise wir die momentane Konzentration in der zweiten Analyseneinheit 38 bestimmt und entsprechende Information wird kontinuierlich zu der Berechnungseinheit 40 übertragen.

Die erste Analyseneinheit 36 umfaßt zumindest ein Filter 42, um die Signalfrequenzen herauszufiltern, die zur Bestimmung des Flusses am geeignetsten sind. Die gefilterten Signale werden zu einer ersten Bestimmungseinheit 46 übertragen, in der die eigentliche Flußbestimmung erfolgt. Die erste Bestimmungseinheit 46 kann dazu auch ein Referenzsignal von der Berechnungseinheit 40 empfangen, das beispielsweise die Frequenz des abgegebenen akustischen Signals angibt. Ausgehend von dem abgegebenen akustischen Signal und dem gemessenen akustischen Signal kann der Einfluß der strömenden Gasmischung auf das akustische Signal und dadurch auch der Fluß bestimmt werden.

Auf entsprechende Weise umfaßt die zweite Analyseneinheit 38 ein zweites Bandpaßfilter 48, um die Frequenzen in dem Meßsignal herauszufiltern, die für die Konzentrationsbestimmung von Interesse sind. Diese Signale werden zu einer zweiten Bestimmungseinheit 50 überführt, in der die Konzentration des spezifischen Gases bestimmt wird. Auch hier kann ein Referenzsignal über die Berechnungseinheit 40 zu der zweiten Bestimmungseinheit 50 überführt werden. Die Berechnungseinheit 40 kann auch als Steuereinheit für den akustischen Sender 26 dienen.

Es sollte hier angemerkt werden, daß der akustische Sender 26 so ausgebildet sein kann, daß er gleichzeitig oder nacheinander eine Mehrzahl unterschiedlicher Frequenzen abgibt, und daß der akustische Empfänger in entsprechender Weise so ausgebildet ist, daß er eine Mehrzahl unterschiedlicher Frequenzen gleichzeitig oder nacheinander mißt. Der akustische Sender 26 beziehungsweise der akustische Empfänger 28 kann zu diesem Zweck mehrere piezoelektrische Elemente zum Erzeugen der unterschiedlichen Frequenzen enthalten.

In Fig. 4 wird ein zweites Ausführungsbeispiel der Meßvorrichtung 22 gezeigt. In diesem Fall sind der erste akustische Sender 26 und akustische Empfänger 28 auf gegenüberliegenden Seiten der Meßkammer 23 angeordnet und das Meßsignal 32 wird diagonal durch die Meßkammer 23 gesendet. Auch hier kann das Signal dazu gebracht werden, ein- oder mehrmals an den Wänden der Meßkammer 23 reflektiert zu werden.

Um die Genauigkeit vor allem bei der Bestimmung der Konzentration der zugeführten Gaskomponente zu erhöhen, kann eine identische Meßvorrichtung auf der Inspirationsseite des Ventilatorsystems angeordnet sein, wie aus Fig. 1 mit der zusätzlichen Meßvorrichtung 14 hervorgeht. Hierdurch wird ein zusätzliches Referenzsignal für die Gasmischung erhalten, die dem Patienten zugeführt wird. Aus dieser Referenz kann dann die Bestimmung der Konzentration des spezifischen Gases auf einfachere Weise sicher erfolgen.

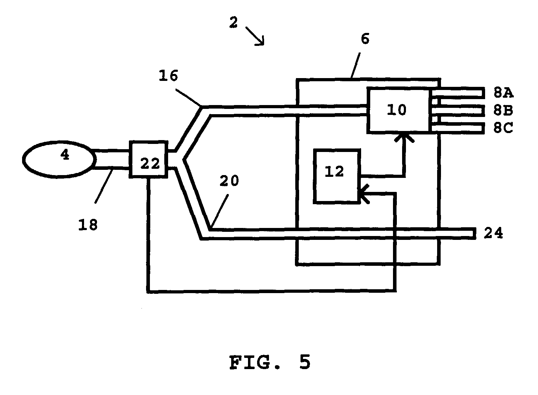

In Fig. 5 wird eine alternative Anordnung der Meßvorrichtung 22 gezeigt, nämlich in dem Patientenschlauch 18. Das ergibt mehrere Vorteile, teilweise können Fluß und Konzentration sowohl während der Inspiration als auch während der Exspiration mit ein und derselben Meßvorrichtung bestimmt werden. Außerdem werden diese Parameter nahe dem Patienten 4 bestimmt, was bedeutet, daß beispielsweise die Kohlendioxidproduktion im Patienten schnell und sicher durch Integrieren des Produktes von gemessenem Fluß und gemessener Konzentration während der Exspirationsphase bestimmt werden kann. Diese und andere Bestimmungen können in der Berechnungseinheit 40 gemacht werden, beispielsweise die Bestimmung des maximalen Flusses während Inspiration/Exspiration, eingeatmetes bzw. ausgeatmetes Volumen, maximale Konzentration des spezifischen Gases, endexpiratiorische Konzentration des spezifischen Gases u.s.w.

Kombinationen der gezeigten Ausführungsbeispiele können, wo angebracht, erfolgen.

## Patentansprüche

1. Meßvorrichtung (22) zum gleichzeitigen Bestimmen des Flusses einer strömenden Gasmischung und der Konzentration eines spezifischen Gases in der Gasmischung mit einer Meßkammer (23), durch die die Gasmischung strömen kann, einem akustischen Sender (26) zum Erzeugen und Abgeben eines akustischen Signals durch die Meßkammer (23), einem akustischen Empfänger (28), um ein übertragenes akustisches Signal zu messen, und einer ersten, an den akustischen Empfänger (28) angeschlossenen Analyseneinheit (36), um den Gasfluß durch die Meßkammer (23) aus dem Einfluß des Gasflusses auf das akustische Signal zu bestimmen, **gekennzeichnet** durch eine zweite, an den akustischen Empfänger (28) angeschlossene Analyseneinheit (38), um die Konzentration des spezifischen Gases in der Gasmischung durch Bestimmen des Einflusses des spezifischen Gases auf das akustische Signal zu bestimmen.

2. Meßvorrichtung nach Anspruch 1, **dadurch gekennzeichnet,** daß die erste Analyseneinheit (36) und/oder die zweite Analyseneinheit (38) an den akustischen Sender (26) angeschlossen sind, um ein Referenzsignal für das abgegebene akustische Signal zu erhalten.

3. Meßvorrichtung nach Anspruch 1 oder 2, **dadurch gekennzeichnet,** daß die zweite Analyseneinheit (38) so ausgebildet ist, daß sie die Konzentration des spezifischen Gases in der Gasmischung durch Bestimmen der Dämpfung der Amplitude des übertragenen akustischen Signals bestimmt.

4. Meßvorrichtung nach Anspruch 3, **dadurch gekennzeichnet,** daß der akustische Sender (26) so ausgebildet ist, daß er nacheinander oder gleichzeitig Signale mit einer Mehrzahl unterschiedlicher Frequenzen erzeugt und abgibt, und daß die Analyseneinheiten (36, 38) so ausgebildet sind, daß sie aus den von dem akustischen Empfänger (28) gemessenen übertragenen akustischen Signalen eine oder mehrere spezifische Frequenzen zum Bestimmen des Flusses bzw. der Konzentration herausfiltern.

5. Meßvorrichtung nach Anspruch 4, **dadurch gekennzeichnet,** daß die Analyseneinheiten (36, 38) Bandpaßfilter (42, 48) umfassen, um die Signale mit den spezifischen Frequenzen herauszufiltern.

6. Meßvorrichtung nach einem der vorhergehenden Ansprüche, **dadurch gekennzeichnet,** daß die Meßkammer (23) so ausgebildet ist, daß das von dem akustischen Sender (26) ausgesendete Signal (32) mehrfach in der Meßkammer (23) reflektiert wird, bevor es auf den akustischen Empfänger (28) trifft.

7. Meßvorrichtung nach einem der vorhergehenden Ansprüche, **dadurch gekennzeichnet,** daß die Meßkammer (23) dazu ausgebildet ist, an eine Exspirationsleitung (20) in einem Ventilator-/Anästhesiesystem (2) angeschlossen zu werden, wobei die Gasmischung aus dem ausgeatmeten Gas besteht, das durch die Meßkammer (23) strömt.

8. Meßvorrichtung nach einem der Ansprüche 1 - 6, **dadurch gekennzeichnet,** daß die Meßkammer (23) ein integrales Teil einer Gasleitung (18) in einem Ventilator/Anästhesiesystem (2) bildet, wobei die Gasmischung aus dem ein- und/oder ausgeatmeten Gas besteht, das durch die Meßkammer (23) strömt.

9. Meßvorrichtung nach einem der Ansprüche 7 - 9, **dadurch gekennzeichnet,** daß die zweite Analyseneinheit (38) die Konzentration von CO₂ in dem ausgeatmeten Gas bestimmt.

10. Meßvorrichtung nach einem der Ansprüche 7 - 9, **gekennzeichnet** durch eine an die erste Analyseneinheit (36) und an die zweite Analyseneinheit (38) angeschlossene Berechnungseinheit (40), um eine Anzahl zu dem ausgeatmeten Gas in Beziehung stehenden Parameter wie der maximal ausgeatmete Fluß, das ausgeatmete Totalvolumen, das Volumen des spezifischen Gases und die maximale Konzentration des spezifischen Gases zu bestimmen.
